# FASCICULE DE BREVET EUROPEEN

(11) **EP 3 256 055 B1**
(45) Date de publication et mention de la délivrance du brevet: **03.08.2022**
(21) Numéro de dépôt: 16707915.1
(22) Date de dépôt: 12.02.2016
(51) Int. Cl.: A61B 17/064, A61B 17/068

(54) **AGRAFE CHIRURGICALE PRESENTANT DEUX BRANCHES MOBILES RELIEES PAR UNE ZONE DE LIAISON TRANSVERSALE**
CHIRURGISCHE KLAMMER MIT ZWEI BEWEGLICHEN ARMEN, DIE DURCH EINEN QUERVERBINDUNGSBEREICH VERBUNDEN SIND
SURGICAL STAPLE HAVING TWO MOVABLE ARMS CONNECTED BY A TRANSVERSE CONNECTION AREA

(30) Priorité: 13.02.2015 FR 1551195
(43) Date de publication de la demande: 20.12.2017
(73) Titulaire: TAURUS ENDOSCOPY, 67000 Strasbourg (FR)
(72) Inventeur: ALZAGA, Amilcar, 03710 Mexico D.F. (MX); HALVAX, Peter, 7635 Pecs (HU); SWANSTRÖM, Lee L, Portland, Oregon 97210 (US)
(74) Mandataire: IP Trust
(86) Numéro de dépôt international: PCT/FR2016/050328
(87) Numéro de publication internationale: WO 2016/128691

(56) Documents cités:
- WO-A1-02/19920
- US-A- 2 684 070
- US-A- 4 610 251
- US-A1- 2008 173 693
- US-A1- 2011 144 691

## Description

### Domaine de l'invention

La présente invention concerne le domaine de la chirurgie endoscope et plus précisément des agrafes pour la suture et la ligature internes ainsi que la pose d'implants fixées sur un tissu par des agrafes. Les agrafes métalliques (le plus souvent en inox) sont utilisées en chirurgie endoscopique ou laparoscopique pour maintenir les deux lèvres tissulaires en contact ou pour la fixation d'une prothèse telle qu'un stent. Elles doivent présenter un encombrement en position déployé compatible avec le passage dans le tube endoscopique et sont manipulées avec un applicateur placé au bout du tube endoscopique. Contrairement aux agrafes destinées à une application cutanée, osseuse ou oculaire, les agrafes endoscopiques doivent permettre un positionnement et une fermeture dans un espace très réduit avec une faible possibilité de mouvement.

### Etat de la technique

On connaît dans l'état de la technique différentes solutions d'agrafes destinées à une pose endoscopique.

La demande de brevet américain US2011/0144691 propose une agrafe présentant une forme générale en « U » avec une base prolongée par deux jambes en forme de « L ». Les jambes sont pliées pour former un angle d'environ 90°. Elles présentent une portion pointue venant perforer les tissus. Elle présente aussi des structures de compression placée au-dessus de la portion de perforation limitant la profondeur de pénétration de la portion de perforation.

Le brevet américain US4610251 décrit une agrafe externe destinée à réunir les deux bords d'une incision dans la peau d'un patient. Ces agrafes sont destinés à être utilisée dans un applicateur et présentent deux branchées avec des extrémités bifurquées transperçant la peau selon deux angles différents.

La demande de brevet internationale WO0219920 décrit une agrafe intracorporelle présentant une base prolongée par deux jambes pénétrant dans les tissus. La base peut être déformée pour refermer l'agrafe sur une plaie.

Le brevet américain US2684070 décrit une agrafe formée par un fil rigide déformé pour présenter une partie centrale allongée prolongée par des parties pointues.

On connaît aussi la demande de brevet US2008/0173693 décrivant un applicateur pour la pose d'agrafes.

### Inconvénients de l'art antérieur

Les solutions de l'art antérieur concernant des agrafes externes destiné à refermer des plaies cutanées ne sont pas pleinement adaptées à une application endoscopique. Pour des applications endoscopiques, l'agrafe doit présenter une très petite dimension, avec des structures d'une épaisseur maximale de moins d'un millimètre et d'une longueur de quelques millimètres.

Par ailleurs, les agrafes de l'art antérieur impliquent une perforation des lèvres tissulaires, provoquant ainsi une amorce de déchirure. Toutes les agrafes proposées dans l'art antérieur présentent des pointes pénétrant profondément dans les tissus pour en assurer le maintien, ce qui provoque des lésions du matériel cellulaire et des sources d'infection.

Enfin, la pression exercée sur les tissus par les agrafes de l'art antérieur pendant et après la fermeture est mal maitrisée et peut provoquer des dégradations voire une nécrose locale.

### Solution apportée par l'invention

La présente invention vise à remédier à ces inconvénients en proposant une agrafe pour application endoscopique, permettant de manipuler et fixer de manière plus adaptée des tissus et/ou des implants dans un espace réduit accessible par voie endoscopique. La présente invention est définie par la revendication 1 relative à une agrafe chirurgicale et la revendication 18 relative au procédé de fabrication de l'agrafe. Les revendications dépendantes définissent des modes de réalisation préférés.

A cet effet, l'invention concerne selon son acception la plus générale une agrafe chirurgicale présentant deux branches reliées par une zone transversale de liaison, caractérisé en ce que chacune desdites branches présentant une zone de pincement et en outre une extrémité d'accrochage, et en ce que ladite zone de liaison transversale est déformable pour permettre le rapprochement desdites branches mobiles par un pliage autour d'une partie centrale de ladite zone de liaison transversale.

L'agrafe selon l'invention présente deux branches mobiles l'une par rapport à l'autre, chacune des deux branches présentant deux extensions recourbées :
- l'une des extensions présente une extrémité pointue pour assurer une perforation superficielle du tissu en vue de son rapprochement avant son pincement. L'axe longitudinal de cette extension forme de préférence un angle de 45° ± 30° par rapport au plan perpendiculaire à l'axe de symétrie de l'agrafe, passant par les extrémités effilées des deux branches, de façon à venir affleurer la surface du bord du tissu et ne pas pénétrer perpendiculairement à la surface des tissus mais avec un angle favorisant un entraînement des bords des tissus pour rapprocher les deux bords lors du repliement des branches l'une vers l'autre.
- l'autre extension présente un méplat pour assurer, en combinaison avec le méplat de l'extension de la branche opposée, le pincement sans perforation des deux bords de tissus à maintenir.

Ces deux méplats sont configurés pour présenter des surfaces de pincement aplaties venant pincer les deux lèvres de tissus réunies lorsque les deux branches sont rapprochées en position de maintien des tissus.

Au moment de l'application, l'agrafe est ouverte, c'est-à-dire que les deux branches sont écartées, avec un angle pouvant aller de 180° (les deux branches sont alors alignées de part et d'autre de la zone de raccordement) à environ 90°. Elles sont appliquées dans cette position ouverte sur la zone à traiter, de façon à ce que les extrémités pointues pénètrent superficiellement dans les bords des tissus à rapprocher. Ensuite, on déforme l'agrafe afin de rapprocher les branches dont les extensions effilées ont accroché superficiellement les deux lèvres des tissus, ce qui produit l'entraînement des bords des tissus par les deux extensions effilées, jusqu'à ce que les bords se rejoignent et se retrouvent « pincés » entre les méplats des deux extrémités plates.

Afin de permettre l'introduction par un tube endoscopique de section minimale, les deux branches sont initialement alignées.

Les deux surfaces de pincement des méplats forment les zones de pincement constituées des surfaces semi-cylindriques. Les tangentes de chacune des surfaces semi-cylindriques, au point central de la zone de pincement sur les tissus lorsque l'agrafe est refermée, forment entre-elles un angle de moins de 30°. Ces tangentes peuvent entre parallèles, convergentes ou divergentes. Le rayon de courbure de ces surfaces semi-cylindriques est supérieur à 10% de la longueur de la branche, et peut être infini lorsque la surface de pincement est plane.

Le rayon de courbure de chaque surface semicylindrique est supérieur à 10% de la longueur d'une branche.

Pour rapprocher les deux branches, on exerce une déformation mécanique provoquant le repliement des branches par rapport à un axe unique contenu dans le plan de symétrie de l'agrafe et passant pas la zone de jonction des deux branches.

De préférence, lesdites branches sont arquées.

Selon une première variante de réalisation, ladite zone de liaison transversale présente une forme semi-tubulaire et en ce que lesdites branches sont mobiles, par déformation de ladite zone de liaison transversales, entre une position de repos où elles sont coplanaires et opposées symétriquement par rapport à un plan médian passant par l'axe de ladite zone transversale semi-tubulaire, et une position où elles sont rabattues l'une vers l'autre.

Avantageusement, chacune des branches présente une extrémité d'accrochage pointue formant un crochet pointu s'étendant en avant du plan comprenant la branche et l'axe de ladite zone transversale semi-tubulaire.

De préférence, la tangente de ladite extrémité d'accrochage pointue forme un angle compris entre 0° et 70°, et de préférence entre 15° et 60° par rapport au plan comprenant la branche et l'axe de ladite zone transversale.

Selon un mode de réalisation particulier, chacune des branches présente un épaulement définissant une surface de pincement configuré de manière à ce que lesdites surfaces de pincement viennent au contact des surfaces extérieures de la zone tissulaire à fixer, lorsque l'agrafe est refermée.

Avantageusement, ledit épaulement est formé par une patte arquée déformable élastiquement, dont une extrémité est reliée à l'une desdites branches et l'autre extrémité formant ladite surface de pincement.

Selon un deuxième mode de réalisation, chacune des branches présente une extrémité fourchue, formée par une extrémité d'accrochage et une patte recourbée présentant une surface de pincement, lesdites branches étant mobiles, par déformation de ladite zone de liaison transversales, entre une position de repos où elles sont sensiblement alignées et opposées symétriquement par rapport à un plan médian passant par l'axe de ladite zone transversale semi-tubulaire, et une position où elles sont rabattues l'une vers l'autre.

Selon une première variante, la distance entre les extrémités pointues est supérieure à la distance entre les surfaces de pincement lorsque l'agrafe est refermée.

Selon une deuxième variante, la distance entre les extrémités pointues est inférieure à la distance entre les surfaces de pincement lorsque l'agrafe est refermée.

La présente divulgation concerne aussi un applicateur d'une agrafe chirurgicale conforme à l'une au moins des revendications précédentes, ledit applicateur présentant au moins un élément tubulaire d'une section correspondant sensiblement à l'enveloppe transversale de l'agrafe, l'applicateur comportant un organe mobile présentant un moyen de coopération transitoire avec la zone de liaison transversale de l'agrafe pour déplacer l'agrafe relativement audit élément tubulaire et des moyens pour assurer la déformation des jambes de l'agrafe, caractérisé en ce que l'interaction entre ledit moyen de coopération et la zone de liaison transversale est réalisée sur la surface intérieure de la zone de liaison transversale, et en ce que la déformation de l'agrafe est réalisée par l'interaction entre la surface extérieure des jambes et l'extrémité frontale dudit élément tubulaire.

Selon une première variante, l'agrafe est placée en position longitudinale dans un prolongement tubulaire, et articulée pour basculer lors de l'extraction hors de ce prolongement tubulaire.

Selon une deuxième variante, l'agrafe est placée en position transversale, et entraînée par un ergot solidaire d'une tige mobile longitudinalement contre la surface frontale d'un manchon tubulaire.

L'invention concerne également un procédé de fabrication d'une agrafe caractérisé en ce que l'on découpe dans une feuille métallique une configuration présentant deux branches mobiles reliées par une zone de liaison transversale, chacune desdites branches présentant une zone de pincement et une extrémité d'accrochage et en ce que ladite zone de liaison transversale est déformable pour permettre le rapprochement desdites branches mobiles par un pliage autour de l'axe de ladite zone de liaison transversale.

Avantageusement ladite découpe est réalisée avec un outil (notamment un faisceau laser ou un jet d'eau) incliné de manière constante pendant la trajectoire de découpe, avec un angle compris entre 10 et 55°.

### Description détaillée d'exemples non limitatifs de réalisation

L'invention sera mieux comprise à la lecture de la description qui suit, se référant aux dessins annexés correspondant à des exemples non limitatifs de réalisation, où :
- la figure 1 représente une vue en perspective d'une agrafe selon une première variante de réalisation l'invention
- la figure 2 représente une vue de dessus de cette agrafe en position ouverte
- la figure 3 représente une vue de dessus de cette agrafe en position fermée
- la figure 4 représente une vue en perspective d'une agrafe selon une deuxième variante de réalisation l'invention
- la figure 5 représente une vue de dessus de cette agrafe en position fermée.
- la figure 6 représente une vue en perspective d'une agrafe selon une troisième variante de réalisation l'invention
- la figure 7 représente une vue de dessus de cette agrafe en position ouverte
- la figure 8 représente une vue de dessus d'une quatrième variante d'agrafe en position ouverte
- les figures 9 et 10 représentent des vues d'un exemple d'applicateur d'agrafes respectivement pendant la phase d'introduction de l'endoscope et pendant la phase d'agrafage
- la figure 11 représente une vue d'un deuxième exemple d'applicateur d'agrafes

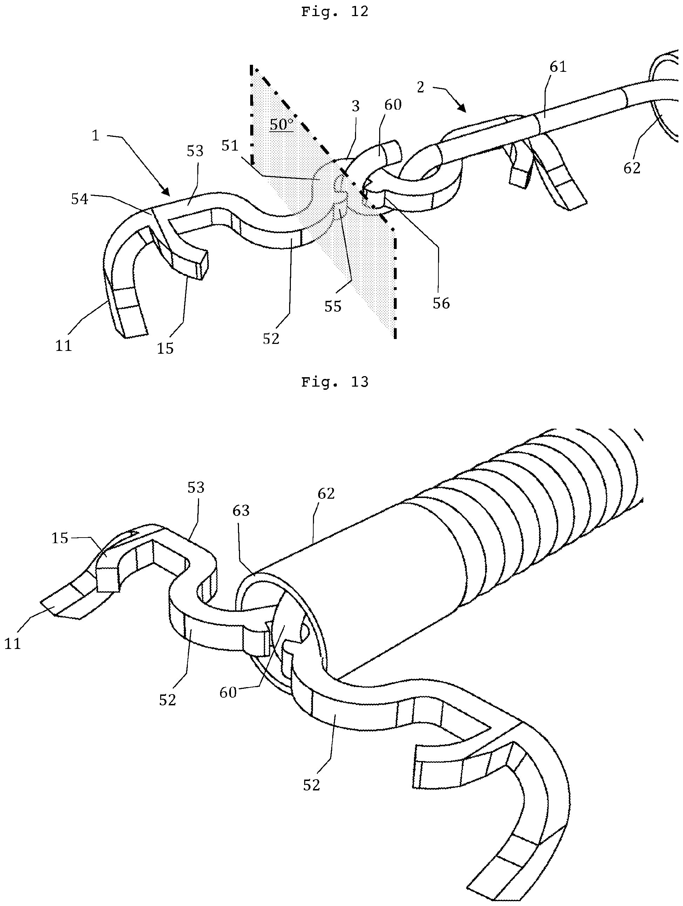

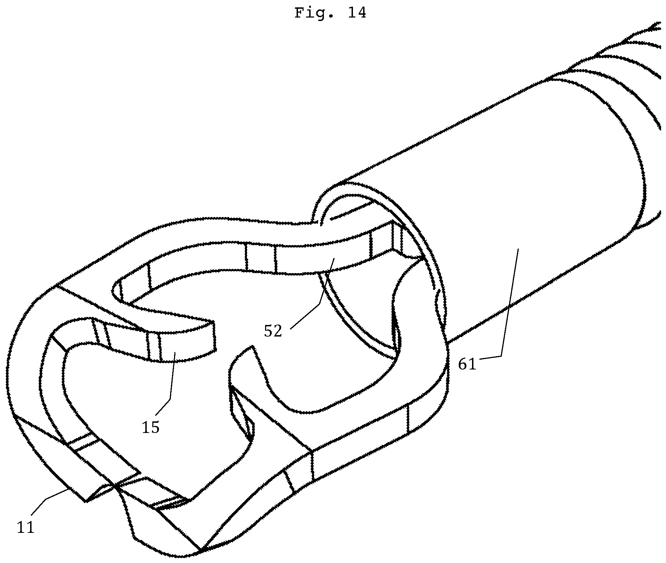

- les figures 12 à 14 représentent des vues d'un autre exemple d'ensemble agrafe / applicateur.

### Description détaillée d'un premier exemple de réalisation d'une agrafe

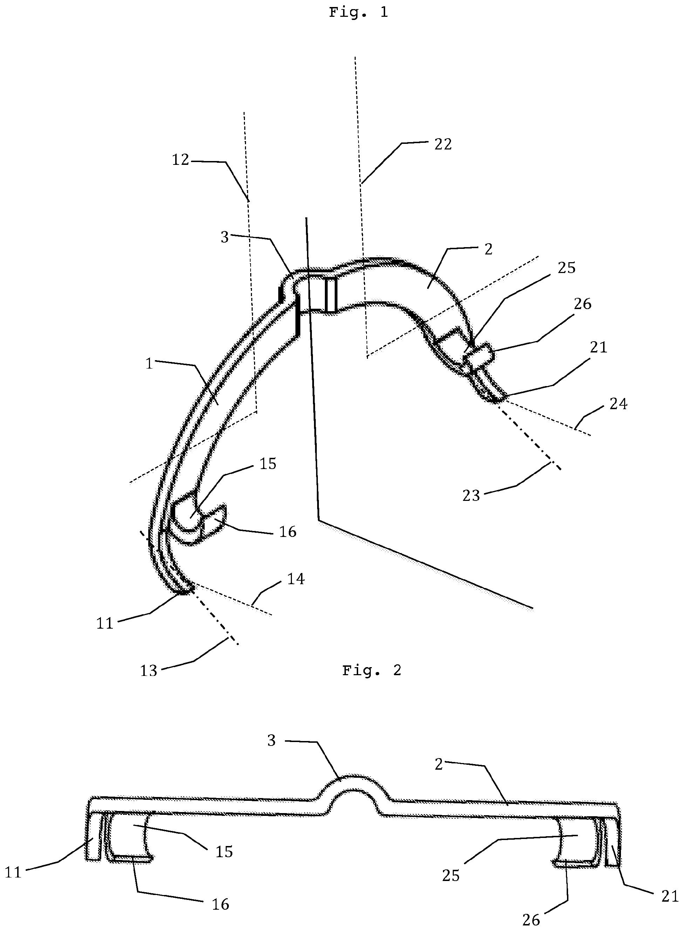

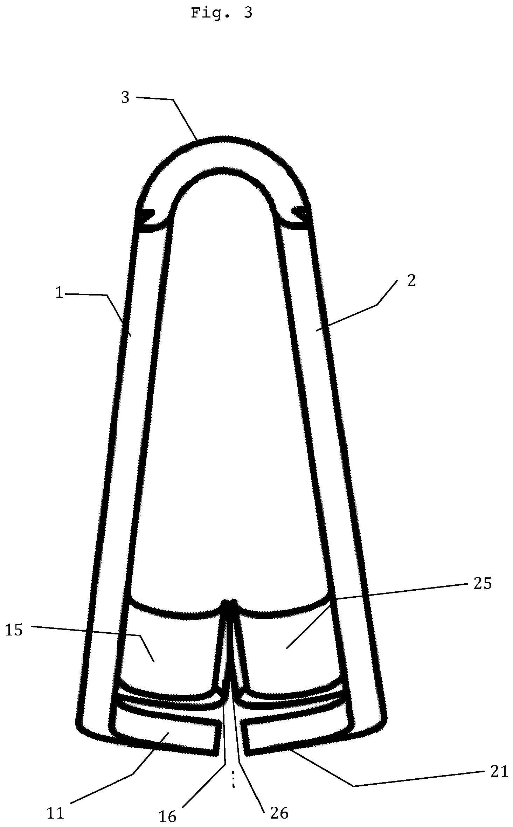

Les figures 1 à 3 représente des vues d'un premier exemple de réalisation d'une agrafe selon l'invention.

L'agrafe présente une forme, en vue de face une forme générale semi-annulaire ou de « fer à cheval ». Dans l'exemple décrit, l'agrafe est formée par une découpe en acier ou en titane mais elle peut aussi être formée par mise en forme d'un fil métallique ou en un matériau biodégradable. Dans cet exemple préféré, le matériau est rigide et déformable avec une faible élasticité et sans mémoire de forme, de façon à conserver la forme imposée lors de la déformation pour le rapprochement des branches.

Elle présente deux branches arquées (1, 2) s'étendant symétriquement par rapport à un plan médian (3) passant par le milieux d'un tronçon de liaison (3). Le tronçon médian (3) désigne simplement la partie comprise entre les deux branches (1, 2). Il n'y a pas de séparation entre cette zone qualifiée de tronçon de liaison (3) et les branches (1, 2) dans l'exemple décrit. Mais il est envisageable dans d'autres formes de réalisation de prévoir une zone (3) reliée par des charnières ou des lignes de pliage aux branches (1, 2).

Ce tronçon médian (3) présente une forme semi-tubulaire et est réalisée par déformation du matériau vers l'arrière avec une matrice cylindrique. La forme semi-tubulaire du tronçon médian (3) permet le positionnement d'un organe de guidage prévu à l'extrémité d'un instrument endoscopique, afin de faciliter la manipulation de l'agrafe au moment de sa pose.

Chacune des deux branches (1, 2) présente une extrémité pointue (11, 21) en forme de crochet, s'étendant en avant du plan transversal (12, 22) contenant la branche correspondante (1, 2).

La tangente (13, 23) à l'extrémité pointue (11, 21) forme, par rapport à la normale (14, 24) au plan transversal (12, 22), un angle compris supérieur à 0° et inférieur à 90°, et de préférence compris entre 5° et 50°. Cette extrémité pointue permet d'accrocher les tissus au voisinage de la zone de pose de l'agrafe, et à exercer une traction latérale pour les rapprocher avant l'agrafage. Lorsque l'angle formé par la tangente est important, on favoriser l'entraînement des tissus sans les perforer. Si l'angle est plus faible, on favorise la pénétration de l'extrémité pointue dans les tissus.

Les deux branches (1, 2) porte également, chacune, un crochet (15, 25) se projetant en avant du plan transversal (12, 22), et positionnés plus près de l'extrémité pointue (11, 21) que de la zone de liaison transversale (3).

L'extrémité pointue d'accrochage (11, 21) et le crochet (15, 25) peuvent être réalisées pour former une extrémité fendue de la branche (1, 2), une des langues de cette extrémité fendue formant la pointe d'accrochage (11, 21) et l'autre formant le crochet (15, 25).

Dans l'exemple décrit, la longueur déroulée de la pointe d'accrochage (11, 21) et sensiblement égale à la longueur déroulée du crochet (15, 25).

Ce crochet (15, 25) présente une surface de pincement (16, 26) parallèle au plan transversal (12, 22). Cette surface de pincement (16, 26) viendra s'appuyer de part et d'autre des tissus au moment de l'agrafage, pour assurer leur maintien sans les perforer.

Le fonctionnement de l'agrafe est le suivant : l'agrafe est positionnée contre les deux lèvres à agrafer avec un applicateur. L'agrafe est en position ouverte, et les deux extrémités pointues (11, 21) viennent affleurer les tissus de part et d'autre de la ligne de séparation des deux lèvres. Ces extrémités pointues (11, 21) pénètre légèrement dans les tissus et les accrochent pour assurer leur rapprochement lorsque l'on commence à refermer l'agrafe par un instrument faisant plier les deux branches (1, 2) par rapport à l'axe médian passant par la partie tubulaire de la zone transversale (3). Les extrémités pointues (11, 21) effectuent alors un mouvement de balayage en arc de cercle, ce qui ramène les tissus au bord des lèvres entre les deux branches, entre les deux crochets (15, 25). Lorsque les deux branches sont rabattues l'une contre l'autre, les surfaces de pincement (16, 26) maintiennent les bords des lèvres en position.

Le fonctionnement est le suivant. L'agrafe est avant utilisation en position ouverte, c'est-à-dire que les branches sont écartées l'une de l'autre. Elles peuvent être, dans certaines réalisation, alignées l'une dans le prolongement de l'autre, de façon à faciliter l'introduction de l'agrafe dans un tube endoscopique de faible section.

Lorsque les extrémités pointues (11, 21) viennent en contact avec les lèvres de tissu à rapprocher, elles pénètrent très superficiellement dans les tissus, pour former deux points d'ancrage. Lorsque l'on applique un effort pour déformer l'agrafe et rapprocher les deux branches (1, 2), ces « ancrages » permettent d'entraîner les lèvres du tissu pour les rapprocher et faire remonter les bords des lèvres dans l'ouverture formée entre les deux branches (1, 2) de l'agrafe. Ces bords sont ramenés dans le plan médian de l'agrafe et viennent au contact de la surface semi-tubulaire (25, 26) non pointue des deux crochets (15, 25) qui ne pénètrent pas dans les tissus, mais viennent exercer une pression selon des directions opposées et pincer les tissus. Lorsque les branches (1, 2) sont repliées dans une position sensiblement parallèle l'une part rapport à l'autre, les surfaces de pincement des tissus sont sensiblement parallèles et exercent une pression perpendiculaire à la surface des tissus, ce qui assure le maintien des deux bords des tissus l'un contre l'autre.

### Description détaillée d'un deuxième exemple de réalisation d'une agrafe

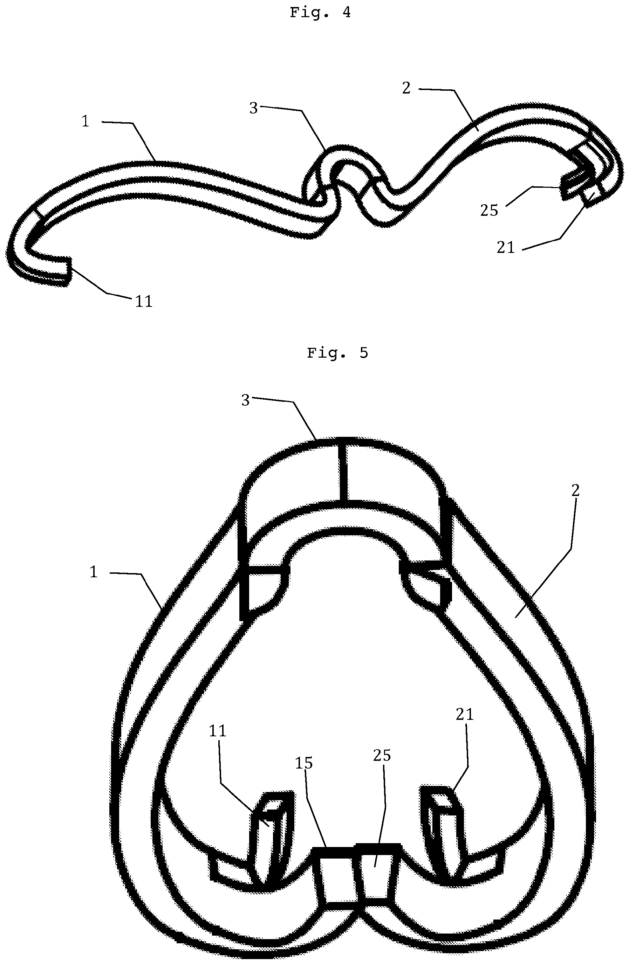

Les figures 4 et 5 représentent une autre variante de réalisation, où les branches (1, 2) forment un « V » dont l'ouverture se réduit lors de la fermeture.

Elle peut être réalisée par pliage d'une tige métallique pour former deux branches arquées (1, 2) terminées chacune par une extrémité fourchue définissant une extrémité d'accrochage (11, 21) et un crochet (15, 25) de pincement des tissus lorsque l'agrafe est refermée.

### Description détaillée d'un troisième exemple de réalisation d'une agrafe

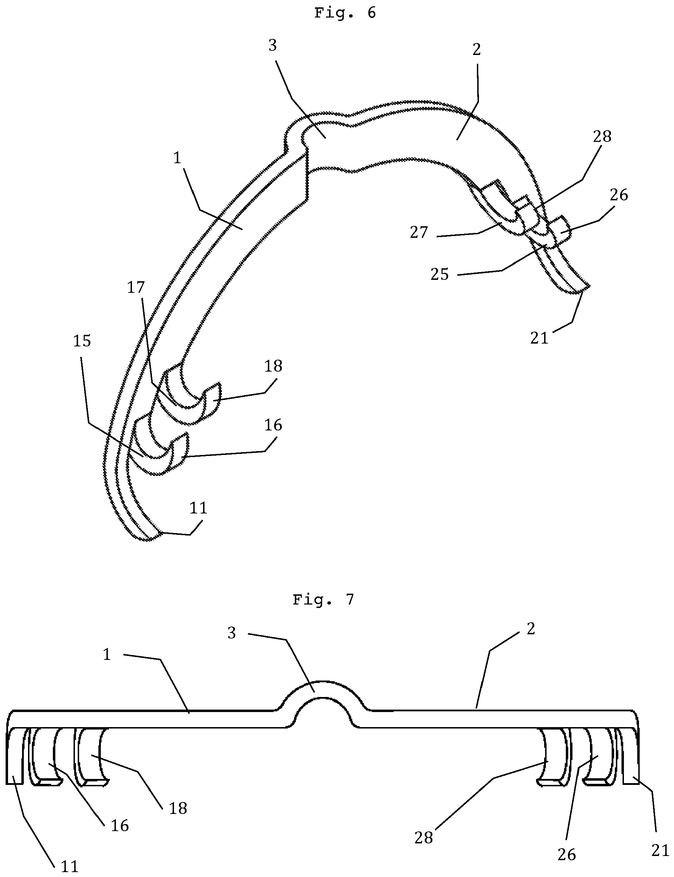

Les figures 6 et 7 représentent une autre variante de réalisation, où les branches (1, 2) portent plusieurs crochets (15, 17 ; 25, 27) présentant chacun une surface de pincement (16, 18 ; 26, 28).

Cette solution permet d'augmenter la surface de pincement des tissus.

### Description détaillée d'un quatrième exemple de réalisation d'une agrafe

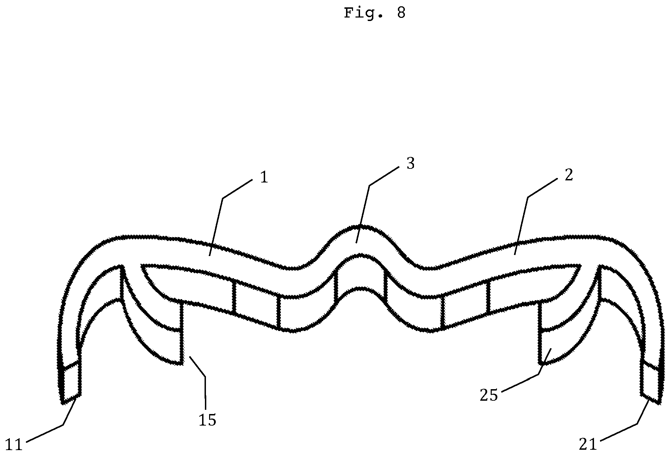

La figure 8 représente une autre variante de réalisation, d'une agrafe découpée dans une plaque métallique par une découpe laser ou une découpe par jet d'eau. La direction de coupe est soit perpendiculaire au plan de la plaque métallique, ou inclinée d'un angle compris entre 10 et 55° constant pendant toute la trajectoire de la découpe, afin de former une agrafe présentant des faces inclinées facilitant le chargement sur l'applicateur.

L'agrafe présente une épaisseur constante et une configuration présentant deux bras (1, 2) s'étendant de part et d'autre d'une zone incurvée, chaque bras (1, 2) présentant une première griffe respectivement (11, 21) et un crochet respectivement (15, 25).

### Description détaillée d'un premier exemple d'applicateur

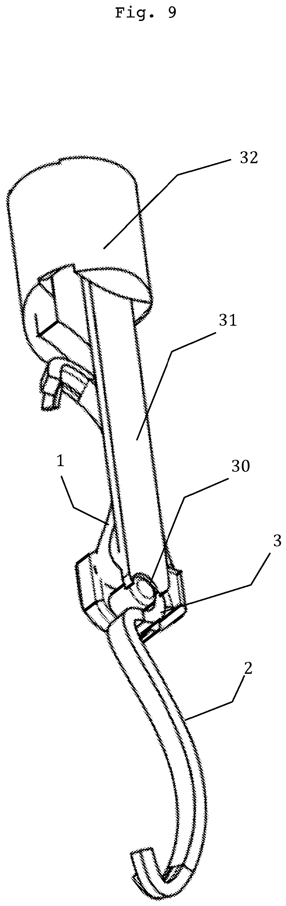

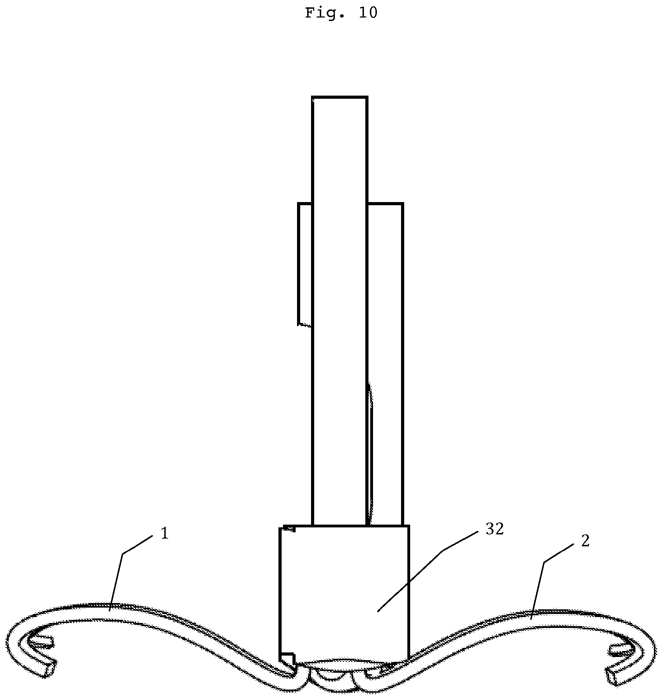

Les figures 9 et 10 présentent un premier exemple d'applicateur endoscopique, pour une agrafe correspondant au deuxième exemple de réalisation. En position d'introduction de l'endoscope, l'agrafe est orientée longitudinalement, de telle sorte que les deux branches (1, 2) sont alignées avec l'axe de l'extrémité tubulaire de l'endoscope. Le segment semicirculaire (3) prolongé de part et d'autre par les branches (1, 2) est positionné autour d'un ergot cylindrique (30) porté par une tige (31) mobile longitudinalement et actionnée par un organe de commande extérieur. Lors de la pose de l'agrafe, l'opérateur repousse la tige (31) afin de faire ressortir la totalité de l'agrafe hors du manchon tubulaire (32) prévu à l'extrémité de l'endoscope. L'étape suivant consiste à retirer la tige mobile (31), ce qui provoque le basculement de l'agrafe dont le dos vient en contact avec la surface frontale du manchon tubulaire (32). L'agrafe vient alors des positionner transversalement, après avoir basculé de 90°, et les deux branches (1, 2) viennent en contact avec la surface frontale du manchon (32), comme représenté en figure 9.

Lorsque l'on continue à exercer une traction sur l'organe de commande de la tige (31), on provoque la déformation de l'agrafe et le rapprochement des deux branches (1, 2), et en conséquence l'accrochage des tissus par les extrémités pointues (11, 21), puis leur pincement par les crochets (15, 25).

### Description détaillée d'un deuxième exemple d' applicateur

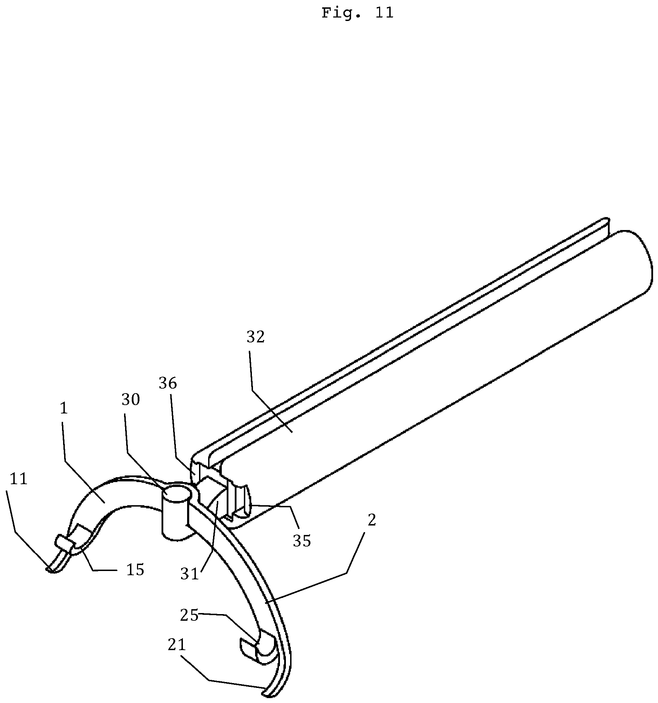

La figure 11 représente un autre exemple de réalisation d'un applicateur. Dans cet exemple, les agrafes sont positionnées transversalement, la partie tubulaire de la zone central (3) étant placé en arrière d'un ergot (30) supporté par une tige (31) mobile transversalement et actionnée par un opérateur à partir d'un organe de manipulation extérieur.

Lorsque l'opérateur retire la tige (31) vers l'arrière, l'ergot (30) entraine l'agrafe jusqu'à ce que les surfaces arrières des branches (31, 32) viennent en contact avec l'extrémité frontale du manchon tubulaire (32) prévu à l'extrémité de l'endoscope. Cette extrémité frontale présente deux méplats (35, 36) assurant le calage des branches (1, 2) de l'agrafe. En poursuivant l'effort de traction, l'agrafe est déformée et les deux branches (1, 2) se rapprochent, ce qui entraîne l'accrochage des tissus par les griffes (11, 21), puis le pincement des tissus entre les deux crochets (15, 25).

### Description d'un autre d'ensemble agrafe/ applicateur

L'agrafe présente deux bras (1, 2) qui sont alignés avant la pose. L'agrafe présente entre ces deux bras (1, 2) une zone de liaison transversale (3).

Cette zone de liaison transversale (3) présente une forme générale de « C », symétrique par rapport au plan médian (4) de l'agrafe. Cette zone de liaison transversale (3) présente de part et d'autre du plan médian un tronçon arqué (51) s'étendant sur un angle compris de préférence entre 70 et 85° se prolongeant par un deuxième tronçon arqué (52) avec un angle de courbure en sens inverse s'étendant sur environ 180°. Ce deuxième tronçon arqué (52) est lui-même prolongé par une troisième tronçon (53) perpendiculaire, avant déformation, au plan de symétrie (4). Ce troisième tronçon (53) présente une bifurcation (54) prolongée par un crochet formant la zone de pincement (15, 25) d'une part et par un second crochet, les deux crochets étant incurvés vers l'intérieur de l'agrafe.

La zone de liaison transversale (3) présente des crans (55, 56) renfermant partiellement l'ouverture, de façon à permettre l'engagement d'un crochet déformable (60) prévu à l'extrémité de la tige (61) de l'applicateur.

Afin de permettre l'introduction dans un tube endoscopique (62), l'agrafe est placée longitudinalement, en position ouverte dans laquelle les deux bras sont alignés, l'un des bras (2) étant appliqué contre la tige (61) et l'autre bras (1) étant opposé, dans le prolongement de la tige (61).

L'applicateur permet d'amener l'agrafe jusqu'au point de pose. La tige (61) est alors repoussée pour faire ressortir l'agrafe en dehors de l'extrémité du tube endoscopique, ce qui provoque le basculement de l'agrafe. En retirant ensuite la tige (61), le crochet (60) entraîne la zone de liaison transversale (3) dans l'embout du tube endoscopique (62), jusqu'à ce que la surface arrière des bras (2, 3), au niveau de la deuxième section (52), vient se bloquer contre le bord frontal (63) de l'extrémité du tube endoscopique (62).

En poursuivant la traction sur la tige (61), le deuxième tronçon (52) se déforme, ce qui provoque le rapprochement des extrémités des bras, et particulièrement des deux crochets formant respectivement les zones d'accrochage (11) et les zones de pincement (15, 25).

Lorsque l'agrafe est refermée, le crochet est décroché en augmentant la traction sur la tige, ce qui déforme le crochet qui s'ouvre et se dégage de la zone de liaison transversale (3) de l'agrafe.

## Revendications

1. - Agrafe chirurgicale présentant deux branches mobiles (1, 2) reliées par une zone de liaison transversale (3), ladite zone de liaison transversale (3) étant déformable pour permettre le rapprochement desdites branches mobiles (1, 2) par un pliage autour de l'axe de ladite zone de liaison (3) transversale, **caractérisée en ce que** chacune desdites branches (1, 2) présente une extrémité fourchue, formée par une extrémité d'accrochage (11,21) et une patte recourbée présentant une zone de pincement (15, 25) et **en ce que** ladite zone de pincement (15, 25) est formée par un premier crochet, ladite extrémité d'accrochage (11, 21) étant formée par un second crochet, les deux crochets étant incurvés vers l'intérieur de l'agrafe lorsqu'elle est en position fermée.

2. - Agrafe chirurgicale selon la revendication 1 **caractérisée en ce que** lesdites extrémités d'accrochage (11,21) sont pointues pour permettre une pénétration superficielle dans les tissus à rapprocher.

3. - Agrafe chirurgicale selon la revendication 1 ou 2 **caractérisée en ce que** chacun des crochets formant lesdites zones de pincement (15, 25) présente sur sa face extérieure une forme semi-cylindriques avec une surface de pincement plane.

4. - Agrafe chirurgicale selon la revendication 1 **caractérisée en ce que** lesdites branches (1, 2) sont arquées.

5. - Agrafe chirurgicale selon la revendication 1 **caractérisée en ce que** les deux branches (1, 2) sont alignées avant la pose et reliés par une zone de liaison transversale (3) présentant une forme générale de symétrique par rapport au plan médian (4) de l'agrafe.

6. - Agrafe chirurgicale selon la revendication précédente **caractérisée en ce que** ladite zone de liaison transversale (3) présente de part et d'autre du plan médian un tronçon arqué (51) s'étendant sur un angle compris de préférence entre 70 et 85° se prolongeant par un deuxième tronçon arqué (52) avec un angle de courbure en sens inverse s'étendant sur environ 180°, ce deuxième tronçon arqué (52) étant lui-même prolongé par une troisième tronçon (53) perpendiculaire, avant déformation, au plan de symétrie (4), ledit troisième tronçon (53) présentant une bifurcation (55) prolongée par ledit crochet formant la zone de pincement (15, 25) d'une part et par ledit second crochet formant la zone d'accrochage (11, 21).

7. - Agrafe chirurgicale selon la revendication 4 **caractérisée en ce que** ladite zone de liaison transversale (3) présente une forme semi-tubulaire et **en ce que** lesdites branches (1, 2) sont mobiles, par déformation de ladite zone de liaison transversale, entre une position de repos où elles sont coplanaires et opposées symétriquement par rapport à un plan médian passant par l'axe de ladite zone transversale semi-tubulaire, et une position où elles sont rabattues l'une vers l'autre.

8. - Agrafe chirurgicale selon la revendication précédente **caractérisée en ce que** le crochet formant ladite zone d'accrochage (11, 21) présent sur chacune des branches (1, 2) est pointu et forme un crochet pointu s'étendant en avant du plan comprenant la branche et l'axe de ladite zone transversale semi-tubulaire.

9. - Agrafe chirurgicale selon la revendication précédente **caractérisée en ce que** la tangente de ladite extrémité d'accrochage pointue forme un angle compris entre 0° et 70° par rapport à la normale au plan comprenant la branche et l'axe de ladite zone transversale.

10. - Agrafe chirurgicale selon l'une au moins des revendications précédentes **caractérisée en ce que** chacune des branches (1, 2) présente un épaulement définissant une surface de pincement (16, 26) configuré de manière à ce que lesdites surfaces de pincement (16, 26) viennent au contact des surfaces extérieures de la zone tissulaire à fixer, lorsque l'agrafe est refermée.

11. - Agrafe chirurgicale selon la revendication précédente **caractérisée en ce que** ledit épaulement est formé par une patte arquée déformable élastiquement, dont une extrémité est reliée à l'une desdites branches et l'autre extrémité formant ladite surface de pincement.

12. - Agrafe chirurgicale selon la revendication 1 ou 2 **caractérisée en ce que** chacune des branches mobiles (1, 2) peut être déformée par déformation de ladite zone de liaison transversale (3), entre une position de repos où elles sont sensiblement alignées et opposées symétriquement par rapport à un plan médian (4) passant par l'axe d'une zone transversale semi-tubulaire, et une position où elles sont rabattues l'une vers l'autre.

13. - Agrafe chirurgicale selon la revendication 10 **caractérisée en ce que** la distance entre les extrémités d'accrochage (11, 21) est supérieure à la distance entre les zones de pincement (15,25) lorsque l'agrafe est refermée.

14. - Agrafe chirurgicale selon la revendication 10 **caractérisée en ce que** la distance entre les extrémités d'accrochage (11, 21) est inférieure à la distance entre les zones de pincement (15,25) lorsque l'agrafe est refermée.

15. - Système comprenant un applicateur et des agrafes conformes à l'une au moins des revendications précédentes, **caractérisé en ce que** ledit applicateur présentant au moins un élément tubulaire d'une section correspondant sensiblement à l'enveloppe transversale de l'agrafe, l'applicateur comportant un organe mobile présentant un moyen de coopération transitoire avec la zone de liaison transversale de l'agrafe pour déplacer l'agrafe relativement audit élément tubulaire et des moyens pour assurer la déformation des branches mobiles (1,2) de l'agrafe, **caractérisé en ce que** l'interaction entre ledit moyen de coopération et la zone de liaison transversale est réalisée sur la surface intérieure de la zone de liaison transversale, et **en ce que** la déformation de l'agrafe est réalisée par l'interaction entre la surface extérieure des branches mobiles (1, 2) et l'extrémité frontale dudit élément tubulaire.

16. - Système selon la revendication précédente **caractérisé en ce que** l'agrafe est placée en position longitudinale dans un prolongement tubulaire (32), et articulée pour basculer lors de l'extraction hors de ce prolongement tubulaire.

17. - Système d'une agrafe chirurgicale selon la revendication 15 **caractérisé en ce que** l'agrafe est placée en position transversale, et entraînée par un ergot (30) solidaire d'une tige (31) mobile longitudinalement contre la surface frontale d'un manchon tubulaire (32) .

18. - Procédé de fabrication d'une agrafe conforme à la revendication 1 **caractérisé en ce que** l'on découpe dans une feuille métallique une configuration présentant deux branches mobiles (1, 2) reliées par une zone de liaison transversale (3), chacune desdites branches (1, 2) présente une zone de pincement (15, 25) et une extrémité d'accrochage (11, 21), ladite zone de liaison transversale (3) étant déformable pour permettre le rapprochement desdites branches mobiles (1, 2) par un pliage autour de l'axe de ladite zone de liaison transversale (3).

19. - Procédé de fabrication d'une agrafe selon la revendication 18 caractérisé en ce ladite découpe est réalisée avec un outil incliné de manière constante pendant la trajectoire de découpe, avec un angle compris entre 10 et 55°.

## Patentansprüche

1. Chirurgische Klammer, die zwei bewegbare Schenkel (1, 2) aufweist, die durch eine Querverbindungszone (3) verknüpft sind, wobei die Querverbindungszone (3) zum Ermöglichen der Annäherung der bewegbaren Schenkel (1, 2) durch ein Biegen um die Achse der Querverbindungszone (3) herum verformbar ist, **dadurch gekennzeichnet, dass** jeder der Schenkel (1, 2) ein gegabeltes Ende, das durch ein Befestigungsende (11, 21) ausgebildet ist, und eine gekrümmte Lasche aufweist, die eine Klemmzone (15, 25) aufweist, und dass die Klemmzone (15, 25) durch einen ersten Haken ausgebildet ist, wobei das Befestigungsende (11, 21) durch einen zweiten Haken ausgebildet ist, wobei die zwei Haken in Richtung der Innenseite der Klammer geschwungen sind, wenn sie in einer geschlossenen Position ist.

2. Chirurgische Klammer nach Anspruch 1, **dadurch gekennzeichnet, dass** die Befestigungsenden (11, 21) zum Ermöglichen eines oberflächlichen Eindringens in die anzunähernden Gewebe spitz zulaufend sind.

3. Chirurgische Klammer nach Anspruch 1 oder 2, **dadurch gekennzeichnet, dass** jeder der Haken, die die Klemmzonen (15, 25) ausbilden, auf seiner Außenseite eine halbzylindrische Form mit einer ebenen Klemmoberfläche aufweist.

4. Chirurgische Klammer nach Anspruch 1, **dadurch gekennzeichnet, dass** die Schenkel (1, 2) bogenförmig sind.

5. Chirurgische Klammer nach Anspruch 1, **dadurch gekennzeichnet, dass** die zwei Schenkel (1, 2) vor dem Einsetzen ausgerichtet und durch eine Querverbindungszone (3) verknüpft sind, die bezogen auf die Mittelebene (4) der Klammer eine allgemein symmetrische Form aufweist.

6. Chirurgische Klammer nach dem vorhergehenden Anspruch, **dadurch gekennzeichnet, dass** die Querverbindungszone (3) auf beiden Seiten der Mittelebene einen bogenförmigen Abschnitt (51) aufweist, der sich über einen Winkel vorzugsweise zwischen 70 und 85° erstreckt, der sich durch einen zweiten bogenförmigen Abschnitt (52) mit einem entgegengesetzten Krümmungswinkel verlängert, der sich über ungefähr 180° erstreckt, wobei dieser zweite bogenförmige Abschnitt (52) ebenfalls durch einen dritten Abschnitt (53) verlängert wird, der vor Verformung senkrecht zu der Symmetrieebene (4) steht, wobei der dritte Abschnitt (53) eine Verzweigung (55) aufweist, die einerseits durch den Haken, der die Klemmzone (15, 25) ausbildet, und durch den zweiten Haken verlängert wird, der die Befestigungszone (11, 21) ausbildet.

7. Chirurgische Klammer nach Anspruch 4, **dadurch gekennzeichnet, dass** die Querverbindungszone (3) eine halbröhrenförmige Form aufweist und dass die Schenkel (1, 2) durch Verformung der Querverbindungszone zwischen einer Ruheposition, in der sie koplanar und bezogen auf eine Mittelebene, die durch die Achse der halbröhrenförmigen Querzone verläuft, symmetrisch gegenüberliegend sind, und einer Position bewegbar sind, in der sie zueinander umgebogen sind.

8. Chirurgische Klammer nach dem vorhergehenden Anspruch, **dadurch gekennzeichnet, dass** der Haken, der die Befestigungszone (11, 21) ausbildet, die auf jedem der Schenkel (1, 2) vorhanden ist, spitz zuläuft und einen spitz zulaufenden Haken ausbildet, der sich vor der Ebene erstreckt, die den Schenkel und die Achse der halbröhrenförmigen Querzone umfasst.

9. Chirurgische Klammer nach dem vorhergehenden Anspruch, **dadurch gekennzeichnet, dass** die Tangente des spitz zulaufenden Befestigungsendes einen Winkel zwischen 0° und 70° bezogen auf die Normale zu der Ebene ausbildet, die den Schenkel und die Achse der Querzone umfasst.

10. Chirurgische Klammer nach wenigstens einem der vorhergehenden Ansprüche, **dadurch gekennzeichnet, dass** jeder der Schenkel (1, 2) eine Schulter aufweist, die eine Klemmoberfläche (16, 26) definiert, die so konfiguriert ist, dass die Klemmoberflächen (16, 26) mit den Außenoberflächen der zu fixierenden Gewebezone in Kontakt kommen, wenn die Klammer geschlossen ist.

11. Chirurgische Klammer nach dem vorhergehenden Anspruch, **dadurch gekennzeichnet, dass** die Schulter durch eine elastisch verformbare, bogenförmige Lasche ausgebildet ist, deren eines Ende mit einem der Schenkel verknüpft ist und deren anderes Ende die Klemmoberfläche ausbildet.

12. Chirurgische Klammer nach Anspruch 1 oder 2, **dadurch gekennzeichnet, dass** jeder der bewegbaren Schenkel (1, 2) durch Verformung der Querverbindungszone (3) zwischen einer Ruheposition, in der sie im Wesentlichen ausgerichtet und bezogen auf eine Mittelebene (4), die durch die Achse einer halbröhrenförmigen Querzone verläuft, symmetrisch gegenüberliegend sind, und einer Position verformt werden kann, in der sie zueinander umgebogen sind.

13. Chirurgische Klammer nach Anspruch 10, **dadurch gekennzeichnet, dass** der Abstand zwischen den Befestigungsenden (11, 21) größer als der Abstand zwischen den Klemmzonen (15, 25) ist, wenn die Klammer geschlossen ist.

14. Chirurgische Klammer nach Anspruch 10, **dadurch gekennzeichnet, dass** der Abstand zwischen den Befestigungsenden (11, 21) kleiner als der Abstand zwischen den Klemmzonen (15, 25) ist, wenn die Klammer geschlossen ist.

15. System, das einen Applikator und Klammern nach wenigstens einem der vorhergehenden Ansprüche umfasst, **dadurch gekennzeichnet, dass** der Applikator wenigstens ein röhrenförmiges Element mit einem Bereich aufweist, der im Wesentlichen der Querumhüllung der Klammer entspricht, wobei der Applikator ein bewegbares Glied umfasst, das ein Mittel für ein vorübergehendes Zusammenwirken mit der Querverbindungszone der Klammer zum Verschieben der Klammer relativ zu dem röhrenförmigen Element und Mittel zum Sicherstellen der Verformung der bewegbaren Schenkel (1, 2) der Klammer aufweist, **dadurch gekennzeichnet, dass** die Wechselwirkung zwischen dem Mittel für das Zusammenwirken und der Querverbindungszone auf der Innenoberfläche der Querverbindungszone ausgeführt wird, und dass die Verformung der Klammer durch die Wechselwirkung zwischen der Außenoberfläche der bewegbaren Schenkel (1, 2) und dem vorderen Ende des röhrenförmigen Elements ausgeführt wird.

16. System nach dem vorhergehenden Anspruch, **dadurch gekennzeichnet, dass** die Klammer in einer Längsposition in einer röhrenförmigen Verlängerung (32) angeordnet ist und zum Kippen während des Herausziehens aus dieser röhrenförmigen Verlängerung angelenkt ist.

17. System einer chirurgischen Klammer nach Anspruch 15, **dadurch gekennzeichnet, dass** die Klammer in der Querposition angeordnet ist und durch einen Vorsprung (30) getrieben wird, der mit einem Schaft (31) fest verbunden ist, der in Längsrichtung gegen die vordere Oberfläche einer röhrenförmigen Hülse (32) bewegbar ist.

18. Verfahren zum Herstellen einer Klammer nach Anspruch 1, **dadurch gekennzeichnet, dass** aus einer Metallfolie eine Konfiguration ausgeschnitten wird, die zwei bewegbare Schenkel (1, 2) aufweist, die durch eine Querverbindungszone (3) verknüpft sind, wobei jeder der Schenkel (1, 2) eine Klemmzone (15, 25) und ein Befestigungsende (11, 21) aufweist, wobei die Querverbindungszone (3) zum Ermöglichen der Annäherung der bewegbaren Schenkel (1, 2) durch ein Biegen um die Achse der Querverbindungszone (3) herum verformbar ist.

19. Verfahren zum Herstellen einer Klammer nach Anspruch 18, **dadurch gekennzeichnet, dass** das Ausschneiden mit einem Werkzeug ausgeführt wird, das während des Ausschneidverlaufs mit einem Winkel zwischen 10 und 55° kontinuierlich geneigt ist.

## Claims

1. Surgical staple having two movable arms (1, 2) which are connected by a transverse connection region (3), said transverse connection region (3) being deformable so as to bring together said movable arms (1, 2) by folding around the axis of said transverse connection region (3), **characterized in that** each of said arms (1, 2) has a forked end that is formed by a hooking end (11, 21) and a curved tab having a gripping region (15, 25) and **in that** said gripping region (15, 25) is formed by a first hook, said hooking end (11, 21) being formed by a second hook, the two hooks being curved toward the inside of the staple when it is in the closed position.

2. Surgical staple according to claim 1, **characterized in that** said hooking ends (11, 21) are pointed so as to penetrate into the surface of the tissues to be brought together.

3. Surgical staple according to either claim 1 or claim 2, **characterized in that** each of the hooks which form said gripping regions (15, 25) has on its outer surface a semicylindrical shape having a planar gripping surface.

4. Surgical staple according to claim 1, **characterized in that** said arms (1, 2) are arcuate.

5. Surgical staple according to claim 1, **characterized in that** the two arms (1, 2) are aligned before being applied and are connected by a transverse connection region (3) having a generally symmetrical shape with respect to the median plane (4) of the staple.

6. Surgical staple according to the preceding claim, **characterized in that** said transverse connection region (3) has on either side of the median plane an arcuate portion (51) which extends over an angle preferably between 70 and 85° and is extended by a second arcuate portion (52) having an angle of curvature in the opposite direction which extends over approximately 180°, this second arcuate portion (52) being itself extended by a third portion (53) which is perpendicular, before deformation, to the plane of symmetry (4), said third portion (53) having a bifurcation (55) which is extended by said hook that forms the gripping region (15, 25) and by said second hook that forms the hooking region (11, 21).

7. Surgical staple according to claim 4, **characterized in that** said transverse connection region (3) has a semi-tubular shape and **in that** said arms (1, 2) are movable, by deformation of said transverse connection region, between a rest position where they are coplanar and symmetrically opposite with respect to a median plane passing through the axis of said semi-tubular transverse region, and a position where they are folded toward each other.

8. Surgical staple according to the preceding claim, **characterized in that** the hook which forms said hooking region (11, 21) on each of the arms (1, 2) is pointed and forms a pointed hook that extends in front of the plane comprising the arm and the axis of said semi-tubular transverse region.

9. Surgical staple according to the preceding claim, **characterized in that** the tangent of said pointed hooking end forms an angle between 0° and 70° with respect to the normal to the plane comprising the arm and the axis of said transverse region.

10. Surgical staple according to at least one of the preceding claims, **characterized in that** each of the arms (1, 2) has a shoulder which defines a gripping surface (16, 26) that is configured in such a way that said gripping surfaces (16, 26) come into contact with the outer surfaces of the tissue region to be fixed when the staple is closed.

11. Surgical staple according to the preceding claim, **characterized in that** said shoulder is formed by an elastically deformable arcuate tab of which one end is connected to one of said arms and the other end forms said gripping surface.

12. Surgical staple according to either claim 1 or claim 2, **characterized in that** each of the movable arms (1, 2) can be deformed by deformation of said transverse connection region (3), between a rest position where they are substantially aligned and symmetrically opposite with respect to a median plane (4) passing through the axis of a semi-tubular transverse region, and a position where they are folded toward each other.

13. Surgical staple according to claim 10, **characterized in that** the distance between the hooking ends (11, 21) is greater than the distance between the gripping regions (15, 25) when the staple is closed.

14. Surgical staple according to claim 10, **characterized in that** the distance between the hooking ends (11, 21) is smaller than the distance between the gripping regions (15, 25) when the staple is closed.

15. System comprising an applicator and staples in accordance with at least one of the preceding claims, **characterized in that** said applicator has at least one tubular element of a section corresponding substantially to the transverse casing of the staple, the applicator comprising a movable member having a means for transiently engaging with the transverse connection region of the staple in order to move the staple with respect to said tubular element and means for deforming the movable arms (1, 2) of the staple, **characterized in that** the interaction between said engaging means and the transverse connection region is carried out on the inner surface of the transverse connection region, and **in that** the staple is deformed by the interaction between the outer surface of the movable arms (1, 2) and the front end of said tubular element.

16. System according to the preceding claim, **characterized in that** the staple is placed in the longitudinal position in a tubular extension (32) and is hinged so as to tilt when extracted from this tubular extension.

17. Surgical staple system according to claim 15, **characterized in that** the staple is placed in the transverse position and moved by a lug (30) which is secured to a rod (31) that is longitudinally movable against the front surface of a tubular sleeve (32).

18. Method for producing a staple according to claim 1, **characterized in that** a configuration having two movable arms (1, 2), which are connected by a transverse connection region, is cut from a metal sheet (3), each of said arms (1, 2) having a gripping region (15, 25) and a hooking end (11, 21), said transverse connection region (3) being deformable so as to bring together said movable arms (1, 2) by folding around the axis of said transverse connection region (3).

19. Method for producing a staple according to claim 18, **characterized in that** said cutting is carried out with a tool which is angled in a constant manner during the cutting trajectory at an angle between 10 and 55°.
